# EUROPÄISCHE PATENTSCHRIFT

(11) **EP 1 062 982 B1**
(45) Veröffentlichungstag und Bekanntmachung des Hinweises auf die Patenterteilung: **30.11.2005**
(21) Anmeldenummer: 00250199.7
(22) Anmeldetag: 23.06.2000
(51) Int. Cl.: A61N 1/372, A61B 5/00, A61B 5/0255

(54) **Verfahren zur Datenabfrage bei der Implantatsnachsorge**
A method of requesting data in implant care
Procédé de demande de données dans l'entretien d'un implant

(30) Priorität: 25.06.1999 DE 19930240
(43) Veröffentlichungstag der Anmeldung: 27.12.2000
(73) Patentinhaber: BIOTRONIK Mess- und Therapiegeräte GmbH & Co Ingenieurbüro Berlin, 12359 Berlin (DE)
(72) Erfinder: Kraus, Michael, 91301 Forchheim (DE); Lang, Martin, 91091 Grossenseebach (DE); Lang, Bernhard, 90537 Feucht (DE); Neudecker, Johannes, 91054 Erlangen (DE); Beetz, Klemens, 91054 Erlangen (DE); Nagelschmidt, Axel, 91052 Erlangen (DE); Potschadtke, Jens, 91052 Erlangen (DE)
(74) Vertreter: Eisenführ, Speiser & Partner

(56) Entgegenhaltungen:
- WO-A-00/30529
- US-A- 5 683 432
- US-A- 5 720 770
- US-A- 5 752 976

## Beschreibung

Die Erfindung betrifft ein Verfahren zur Nachsorge bei Patienten mit wenigstens einem elektromedizinischen Implantat gemäß dem Oberbegriff des Anspruchs 1.

Bei Patienten mit wenigstens einem elektromedizinischen Implantat, wie beispielsweise einem Herzschrittmacher, einem Defibrillator, einem Kardioverter oder einem anderen elektronisch betätigten bzw. gesteuerten Implantat, ist es erforderlich eine mehr oder weniger intensive Nachsorge zu betreiben.

Bei bekannten Verfahren erfolgt hierzu in der Regel eine telemetrische erste Datenübertragung zwischen dem Implantat und einem zugeordneten externen Gerät, bei der erste Daten von dem Implantat an das externe Gerät übertragen werden. Anläßlich einer Nachsorgeuntersuchung des Patienten in einer Nachsorgeeinrichtung werden dann zur Information des Untersuchenden im Rahmen einer Nachsor geabfrage wenigstens die aktuellsten zur Verfügung stehenden Daten des Implantats abgefragt und zur Ausgabe an entsprechende Ausgabemittel gesandt.

Derartige Nachsorgeabfragen werden, beispielsweise bei Herzschrittmacherpatienten, üblicherweise mittels kurzreichweitiger Telemetrie durchgeführt. Diese gestaltet sich in der Regel relativ aufwendig und langwierig, da hierbei das externe Gerät unmittelbar auf die Haut des Patienten aufgesetzt werden muß und das externe Gerät über einen relativ langen Zeitraum möglichst genau positioniert bleiben muß. Andererseits ist es, beispielsweise aus der US-Patentschrift US-5,720,770 bekannt, über entsprechende Patientenüberwachungssysteme eine Ferndiagnose durchzuführen. Dies weist jedoch den Nachteil auf, daß der Untersuchende keinen unmittelbaren Kontakt mit dem Patienten hat, sondern diesen, wie in der US 5,720,770 vorgeschlagen, bestenfalls per Telefon befragen kann. Optische Eindrücke und Stimmungsausdrücke, aus denen ein erfahrener Arzt wichtige Rückschlüsse ziehen kann, gehen dabei verloren.

Der Erfindung liegt deshalb die Aufgabe zugrunde, ein Verfahren der eingangs genannten Art zur Verfügung zu stellen, das eine schnelle, einfache und zuverlässige Nachsorgeuntersuchung vor Ort ermöglicht.

Die Aufgabe wird, ausgehend von einem Verfahren gemäß dem Oberbegriff des Anspruchs 1, durch die im kennzeichnenden Teil des Anspruchs 1 angegebenen Merkmale gelöst.

Die Erfindung schließt die technische Lehre ein, eine schnelle, einfache und zuverlässige Nachsorgeuntersuchung vor Ort sicherzustellen, indem die im Rahmen der Nachsorgeabfrage vorgenommene Abfrage der Daten vor der Nachsorgeuntersuchung automatisch nach Eintreffen des Patienten in einem Abfragebereich der Nachsorgeeinrichtung erfolgt. Die Übertragung der Daten erfolgt mittels einer langreichweitigen Telemetrieverbindung zwischen dem Implantat und dem externen Gerät. Alternativ oder zusätzlich kann die Übertragung der Daten mindestens teilweise auch über ein Mobilfunknetz oder ein Telekommunikationsfestnetz erfolgen. Dabei ermöglicht es die über eine große Reichweite erfolgende Datenübertragung, daß sich der Patient in dem Abfragebereich mehr oder weniger frei bewegen kann. Die automatische Abfrage ohne Zutun des Untersuchenden oder etwaigen sonstigen Personals entlastet zudem den untersuchenden Arzt bzw. dessen Personal.

Vorteilhafterweise wird zur weitergehenden Information des Untersuchenden neben der Abfrage der Daten automatisch wenigstens ein in einer zentralen Speichereinrichtung gespeicherter, aus einer vorherigen Datenausgabe des Implantats stammender Datensatz abgerufen und zur Ausgabe an die Ausgabemittel gesandt, so daß sich der untersuchende Arzt unmittelbar ein Bild von der Entwicklung des Patienten machen kann.

Zwecks der besonders gut strukturierten Aufbereitung der Information wird dabei bevorzugt eine nach Art einer Datenbank organisierte zentrale Speichereinrichtung verwendet. Vorzugsweise handelt es sich dabei um eine zentrale Speichereinrichtung mit einem angekoppelten Expertensystem, so daß dem untersuchenden Arzt beispielsweise unmittelbar der Vergleich mit ähnlich gelagerten Fällen zur Verfügung steht.

Zur weitergehenden Information des Arztes können über die telemetrische Datenübertragung Tests, beispielsweise die Reizschwell-Untersuchung, Belastungstests für die Response-Faktor oder Schocktests, oder auch Diagnoseprogramme generiert werden, deren Ergebnisse über die telemetrische Datenübertragung an das externe Gerät übertragen werden.

Zur besseren und einfacheren Verwertbarkeit der Information für den Arzt erfolgt bevorzugt vor der Ausgabe durch die Ausgabemittel eine automatische diagnostische Vorauswertung und alternativ oder zusätzlich eine Aufbereitung der Daten.

Bei dem erfindungsgemäßen Verfahren wird als externes Gerät ein im Abfragebereich installiertes externes Gerät verwendet, wobei die Abfrage der Daten erfolgt, sobald sich das Implantat für einen vorgegebenen Zeitraum in Abfragereichweite zum externen Gerät befindet.

Bei anderen Varianten wird als externes Gerät ein vom Patienten mitgeführtes mobiles externes Gerät verwendet. Dabei kann das externe Gerät unmittelbar an eine entsprechende Abfrageeinrichtung im Abfragebereich angeschlossen und so automatisch die Abfrage ausgelöst werden.

Ebenso ist es möglich ein externes Gerät für eine Fernüberwachung zu nutzen, wobei die Übertragung der Daten dann über das Fernüberwachungssystem erfolgt. Hierbei erfolgt das Auslösen der Abfrage der Daten dann vorzugsweise durch eine im Abfragebereich installierte ersten Auslöseeinrichtung, die mit einer zugeordneten zweiten Auslöseeinrichtung des Implantats und/oder des mobilen externen Geräts zusammenwirkt.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: ein Blockschaltbild eines bevorzugten Ausführungsbeispiels einer Vorrichtung zur Durchführung des erfindungsgemäßen Verfahrens;
- Figur 2: einen Ablaufplan des erfindungsgemäßen Verfahrens;
- Figur 3: ein Detail aus Figur 2;
- Figur 4: ein Blockschaltbild einer Variante eines Patientenüberwachungssystems unter Verwendung der vorliegenden Erfindung;
- Figur 5: ein Blockschaltbild einer Variante eines Patientenüberwachungs- und nachsorgesystems unter Verwendung der vorliegenden Erfindung;
- Figur 6: ein Blockschaltbild einer weiteren Variante eines Patientenüberwachungs- und nachsorgesystems unter Verwendung der vorliegenden Erfindung;
- Figur 7: ein Blockschaltbild einer Telemetrieeinrichtung eines elektromedizinischen Implantats unter Verwendung der vorliegenden Erfindung;
- Figur 8: die Sender/Empfängereinheit einer Variante der Ausführung aus Figur 1;
- Figur 9: x ein Schaltbild eines Senders einer Variante der Ausführung aus Figur 1;
- Figur 10: ein Blockschaltbild einer weiteren bevorzugten Ausführung einer erfindungsgemäßen Vorrichtung.

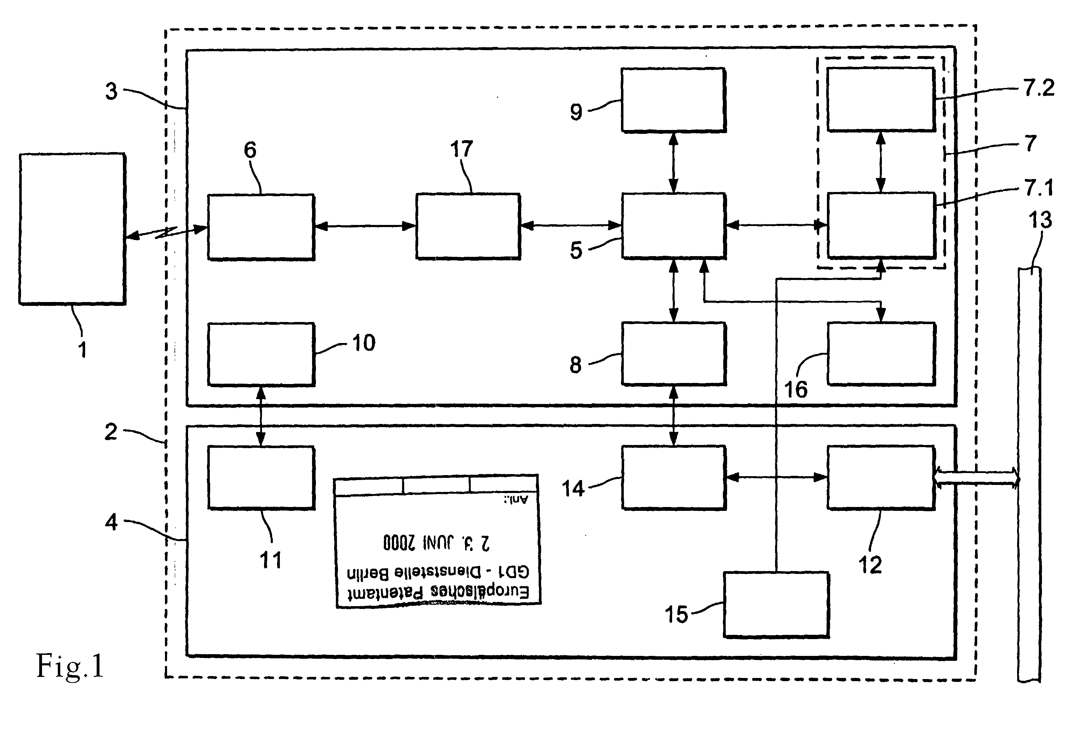

Figur 1 zeigt eine Vorrichtung zur Durchführung des erfindungsgemäßen Verfahrens mit einem elektromedizinischen Implantat 1, das mit einer Telemetrieeinrichtung versehen ist und einem externen Gerät 2, das aus einem Mobilteil 3 und einer Basisstation 4 besteht.

### Mobilteil 3

Das Mobilteil 3 weist dabei eine Steuereinrichtung 5 sowie eine erste Schnittstelleneinrichtung 6, eine Mobilfunkeinrichtung 7, eine zweite Schnittstelleneinrichtung 8, ein Man-Machine-Interface 9 und eine dritte Schnittstelleneinrichtung 16 auf, die jeweils mit der Steuereinrichtung 5 verbunden sind. Die erste Schnittstelleneinrichtung 6 ist mit der Steuereinrichtung 5 indirekt über eine weitere Schnittstelleneinrichtung 17 verbunden. Die Stromversorgung des Mobilteils erfolgt über Li-lonenakkus, die im Stromversorgungsteil 10 untergebracht sind.

Die erste Schnittstelleneinrichtung 6 besteht aus einer Telemetrie-Sender/Empfängereinheit, so daß das Mobilteil 3 über eine bidirektionale Telemetriestrecke Kontakt zum Implantat aufnehmen kann. Empfänger und Sender der Schnittstelleneinrichtung 6 sind in einem IC integriert. Beide Schaltungsteile bilden eine funktionale Einheit. Empfänger und Sender arbeiten vorzugsweise im UHF-Bereich bei 403,55 MHz, als Baudratenstufen sind 4, 8, 16 und 32 KBit/s vorgesehen. Möglich ist aber insbesondere für das Implantat 1 auch die Verwendung energiesparenderer Frequenzen im VHF- oder LF-Bereich.

Die Mobilfunkeinrichtung 7 kann Daten, die beispielsweise über die erste Schnittstelleneinrichtung 6 aus dem Implantat ausgelesen wurden, in Form von SMS-Nachrichten versenden und von einer als Überwachungseinrichtung oder zentrale Speichereinrichtung dienenden Servicezentrale angerufen werden.

Über die zweite Schnittstelleneinrichtung 8 kann eine Verbindung zwischen Mobilteil 3 und Basisstation 4 hergestellt werden. Sie ist als Infrarotschnittstelle (IrDA) ausgebildet. Sie arbeitet bidirektional und kann im halb-duplex-Betrieb Daten übertragen.

Das Man-Machine-Interface 9 ermöglicht eine Bedienung des Mobilteils 3 durch einen Benutzer.

Die dritte Schnittstelleneinrichtung 16 wird für die Aufnahme von externen Daten oder für den Service verwendet. Sie ist ebenfalls als IrDA-Schnittstelle ausgebildet.

Die serielle Schnittstelle für beide IrDA-Schnittstelleneinrichtungen 8 und 16 und die Schnittstelle zur Mobilfunkeinrichtung 7 liegen parallel, so daß immer nur eine Einheit betrieben werden kann. Die IrDA-Schnittstellen 8 und 16 sind parallel geschaltet. Beide Schnittstellen sind dauernd empfangsbereit, so daß jederzeit eine externe Anforderung bedient werden kann. Die Software muß dann lediglich noch feststellen, bei welcher IrDA-Schnittstelle die Empfangsanforderung vorliegt.

Die Stromversorgung des Mobilteils 3 ist mit Li-Ionen-Akkus realisiert. Sie können das Mobilteil 3 im voll aufgeladenen Zustand mindestens 20h (Standbyzeit + 16 SMS-Übertragungen) versorgen. Die Dimensionierung der Akkus gilt für das Lebensdauerende. Es ist deshalb ein 1000 mAh Li-Ionen-Akku vorgesehen.

### Mobilteil 3: Modulare Ausführung

In einer nicht eigens dargestellten weiteren Ausführung ist die Telemetrieeinheit 6 für die Kommunikation mit dem Implantat 1 inklusive einer Sende/Empfangsantenne und einer Ansteuerelektronik für die Telemetrie sowie einer Schnittstelleneinrichtung 17 in einem separaten Modul untergebracht. Dieses separate Modul weist eine Schnittstelleneinrichtung 17 zur elektrischen und gegebenenfalls auch mechanischen Verbindung mit einem Mobiltelefon auf.

Das separate Modul kann beispielsweise anstelle des Batteriepacks in das Mobiltelefon eingesetzt sein, so daß sich eine mechanisch stabile Einheit aus Mobiltelefon und separatem Gehäuse ergibt, welche gegebenenfalls die bisherige äußere Kontur des Mobiltelefons unwesentlich überragt.

Bei dieser Variante weist das separate Modul ein Batteriefach auf, in welches der originale Batteriepack des Mobiltelefons oder aber eine andere geeignete Batterie oder ein geeignetes Batteriepack eingelegt werden kann. Diese Batterie dient dann zur Energieversorgung des Gesamtsystems aus Mobiltelefon und separatem Modul und kann über die serienmäßigen Ladeanschlüsse am Mobiltelefon geladen werden.

Zur Datenverbindung zwischen dem separaten Modul und dem Mobiltelefon gibt es bei dieser Variante drei grundsätzliche Möglichkeiten:
1. über den externen elektromechanischen Stecker der am Mobiltelefon vorhandenen (üblicherweise seriellen) Schnittstelle,
2. über einen zusätzlich im Batteriefach des Mobiltelefons zu integrierenden elektromechanischem Stecker der am Mobiltelefon vorhandenen (seriellen) Schnittstelle oder
3. über die am Mobiltelefon vorhandene IrDA-Schnittstelle.

Bei der 1. und 3. Variante existiert zwischen dem separaten Modul und der im Mobiltelefon integrierten Schnittstelle eine optische oder mechanische Verbindung, d. h. das separate Modul oder ein fester Fortsatz desselben überdeckt die Schnittstelle des Mobiltelefons und enthält an dieser Stelle eine geeignete Steckverbindung oder reicht bis in die Nähe der Schnittstelle des Mobiltelefons und enthält an diesem Ende eine Infrarot-Schnittstelle. Diese Lösungen bedingen eine spezielle Software für das Mobiltelefon, welche eine Steuerung, die über diese Schnittstelle initiiert wird, zuläßt.

Die 2. Variante erfordert zur Integration des Steckers im Batteriefach zusätzlich eine mechanische und elektrische Modifikation des Mobiltelefons. Durch eine geeignete Verbindung dieses Steckers nicht nur mit der seriellen Schnittstelle sondern auch mit anderen Teilen der Elektronik des Mobiltelefons kann eine direkte Steuerung des Mobiltelefons durch die Telemetrieeinheit ermöglicht werden.

In beiden Fallen bleibt die bekannte, normale Funktion des Mobiltelefons erhalten. Gegebenenfalls kann eine Vorrangsteuerung für Telemetrieübertragungen vorgesehen werden. Vorzugsweise kann die Elektronik des Mobiltelefons durch die permanent in Empfangsbereitschaft befindliche oder wahlweise zeitgesteuerte Telemetrieeinheit bei Bedarf automatisch in Betrieb genommen werden (Ereignissteuerung).

In einer weiteren Variante kann das separate Modul auf einer SIM-Karte integriert sein. Dieses SIM-Modul wird anstelle der standardmäßigen SIM-Karte in den SIM-Karten-Einschub gesteckt und verwendet die hierfür standardmäßig vorgesehene Schnittstelle.

Gegebenenfalls können beispielsweise die Sende/Empfangsantenne und die Ansteuerelektronik für die Telemetrie so ausgeführt sein, daß sie bei eingeschobenem SIM-Modul außerhalb des Gehäuses des Mobiltelefons angeordnet sind.

Für die akustische Anzeige der verschiedenen Patienten-Alarm-Funktionen kann bei dieser Variante ein in Mobiltelefonen üblicher Buzzer angesteuert werden. Für die optische Anzeige kann in allen modularen Varianten die an Mobiltelefonen ohnehin vorhandene optische Anzeige genutzt werden.

### Mobilteil 3: Man-Machine-Interface

Am Mobilteil 3 sind eine LED und ein Buzzer zur optischen und akustischen Anzeige von Funktionen und Betriebszuständen sowie einige Schalter zur manuellen Auslösung von Funktionen vorgesehen.

Eine blinkende Leuchtdiode zeigt den Low-Battery-Zustand an, sobald die Akkuladung auf 10% abgesunken ist. Das Mobilteil 3 hat dann noch minimal etwa 2 Stunden Gangreserve. Da das Mobilteil 3 für den Patienten nicht immer sichtbar ist, zeigt der Buzzer durch einen kurzen Alarm ebenfalls das Eintreten des Low-Battery-Zustandes an. Der Buzzer muß auf einer Frequenz von etwa 2 kHz oder darunter arbeiten und entsprechend laut sein, so daß auch in der Wahrnehmung eingeschränkte Patienten den Alarmton sicher hören können. Der Buzzer sollte etwa alle 5 Minuten wieder Alarm auslösen, um den Patienten an das Aufladen des Akkus zu erinnern.

Es ist ein versenkter Ein/Aus-Schalter vorgesehen, der nicht unbeabsichtigt betätigt werden kann. Die Schalterposition ist beschriftet, so daß ersichtlich ist, ob das Mobilteil 3 eingeschaltet ist. Dieser Schalter ist notwendig, da das Mobilteil 3 mit der Mobilfunkeinrichtung 7 in einigen Umgebungsbereichen ausgeschaltet werden muß, z. B. während eines Fluges und in bestimmten Bereichen von Krankenhäusern.

Es kann eine Taste vorgesehen sein, um eine Short Message zum Testen des SMS-Kanals an die Servicezentrale zu senden. Das Mobilteil 3 zeigt durch eine blinkende LED an, daß eine solcher Test gestartet wurde. Die Servicezentrale erkennt die empfangene Short Message als Test und sendet eine Quittung an das Mobilteil 3. Daraufhin wird das Blinken der LED wieder beendet, wodurch für den Patienten erkennbar ist, daß sowohl Hin- als auch Rückverbindung zur Servicezentrale funktionieren.

Für diagnostische Zwecke kann eine Taste vorgesehen sein, mittels derer der Patient Datenübertragungen zur Servicezentrale auslösen kann. Treten beim Patient Beschwerden jeglicher Art auf, die möglicherweise für das Implantat gar nicht als solche erkennbar sind, kann diese Taste gedrückt werden. Daraufhin wird ein Datensatz übertragen, der neben den routinemäßig zu übertragenden Informationen den Zeitpunkt der Auslösung und der Gewinnung der zugehörigen Daten durch das Implantat enthält. Durch die übertragene Short Message wird die Servicezentrale außerdem darüber informiert, daß es sich um eine patientengetriggerte Datenübertragung handelt, die gegebenfalls gesondert zu behandeln ist.

Für akute Notfälle kann eine Patientenalarm-Taste eingesetzt sein, die ebenfalls vor unbeabsichtigter Fehlbedienung geschützt ist. Diese Notfalltaste kann zu diesem Zweck versenkt angeordnet und zusätzlich durch einen Deckel geschützt sein, der erst geöffnet werden muß, um den Patientenalarm auszulösen. Der Fehlbedienungsschutz kann auch als Zeitsperre in der Software ausgeführt sein, welche die Tastenfunktion erst als gültig annimmt, wenn die Taste länger (z.B. mehr als 3 s) gedrückt wird. Ein erfolgreich ausgelöster Patientenalarm wird akustisch durch den Buzzer quittiert. Danach kann die Taste wieder losgelassen werden. Weiterhin ist eine Leuchtdiode für 10s dauereingeschaltet und leuchtet dann jede Minute nochmals für 2s zur Erinnerung an einen ausgelösten Patientenalarm. Der Buzzer wird immer gleichzeitig mit der Leuchtdiode betätigt, so daß auch in der Wahrnehmung eingeschränkte Patienten diese Quittung des externen Geräts wahrnehmen können.

Ein Patientenalarm wird sofort über SMS an die Servicezentrale weitergeleitet. Er wird erst gelöscht, wenn die Servicezentrale den Patientenalarm - ebenfalls über SMS - quittiert und so sichergestellt ist, daß der Alarm in der Servicezentrale angekommen ist. Die Quittierung des Alarms durch die Servicezentrale wird ebenfalls über Leuchtdiode und Buzzer am Mobilteil 3 angezeigt. Diese Maßnahmen tragen einerseits zur Beruhigung des Patienten bei, andererseits wird verhindert, daß bei Auslösung eines Patientenalarms in einem gefährlichen Zustand des Patienten weitere Maßnahmen unterlassen werden, wenn der Patientenalarm nicht erfolgreich übertragen werden konnte.

Soll ein nicht quittierter Patientenalarm gelöscht werden, muß das Mobilteil 3 ausgeschaltet werden. Nach dem Neustart ist das Mobilteil wieder im Normalzustand. Via SMS meldet sich das Mobilteil automatisch in der Servicezentrale zurück. Dort kann dann erkannt werden, daß das externe Gerät zurückgesetzt wurde und der Patientenalarm gelöscht ist.

Die erfolgreiche Durchführung eines Patientenalarms hat gegenüber der Low-Battery-Anzeige eine höhere Priorität. Die Low-Battery-Anzeige wird erst wieder angezeigt, wenn das Servicezentrum den Patientenalarm quittiert hat. Soll eine Störung der Umgebung durch den Buzzer verhindert werden, ist das Mobilteil 3 auszuschalten.

### Mobilteil 3: Datenverschlüsselung

Für das Mobilteil 3 wird nur ein Prozessor als Steuereinheit 5 eingesetzt, der sowohl die Aufgaben der Zentraleinheit, als auch die der Kommunikationseinheit übernimmt. Die Software ist über die IrDA-Schnittstelle 16 in ein Flash-ROM downloadbar. Die Speicherkapazität des internen Flash-ROMs betragt 128 KByte, die des integrierten EEPROMS 4 KByte und die des statischen, externen SRAMs 64 KByte. Die Programmspeichergröße ist somit auf die Kapazität des eingebauten Flash-ROMs begrenzt und kann nicht erweitert werden (Harvard-Struktur). Das externe statische SRAM ist erweiterbar.

Für die SMS-Übertragung wird ein 128 Bit DES Verschlüsselungsverfahren angewendet, das auf einem private-public key System basiert. Die Verschlüsselung wird softwaremäßig realisiert, d.h. es wird kein Verschlüsselungs-IC verwendet. Für einen sicheren Betrieb ist eine Watchdog-Schaltung vorgesehen, die in regelmäßigem Abstand den Prozessor 5 überprüft und ihn wieder in einen sicheren Status überführt, wenn die Programmausführung nicht korrekt abläuft.

Auf dem Weg vom Implantat 1 zum externen Gerät 2 ist wegen der kurzen Reichweite zusätzlich zu der implantatspezifischen Kodierung der Daten keine weitere Verschlüsselung mehr notwendig.

### Mobilteil 3: Ortung

Für die Mobilfunkeinrichtung 7 ist vorgesehen, daß über SMS die Feldstärkeinformation mit den dazugehörigen Nummern der current and adjoined cells und die Bitfehlerrate übertragen wird. Anhand dieser Ortungsinformationen ist eine Ortung des Mobiltels 3 in Notfällen, in denen der Patient handlungsunfähig ist, möglich.

### Mobilteil 3: Rufnummernverwaltung

Die Mobilfunkeinrichtung 7 um faßt ein Mobilfunkmodul 7.1 und einen als SIM-(Subscriber Identity Module-) Karte 7.2 ausgebildeten Speicher. Der SIM-Kartenleser kann unter dem Mobilfunkmodul 7.1 direkt in die Leiterplatte gelötet werden. Dem Anwender ist damit die SIM-Karte 7.2 nicht zugänglich. Bei Ausführungsformen, die auf der Verwendung eines Mobiltelefons basieren, ist ein SIM-Kartenleser ohnehin vorhanden.

Im Speicher der SIM-Karte 7.2 wird bzw. werden durch das Mobilfunkmodul 7.1 die Rufnummer(n) der Servicezentrale permanent abgelegt. Das Mobilteil 3 wird mit einer SIM-Karte ausgeliefert, die im Rufnummernspeicher als Voreinstellung die Rufnummer einer Servicezentrale enthält. Via SMS können die Rufnummern auf der SIM-Karte geändert werden. Die Rufnummernverwaltung (Schreiben und Lesen der Nummern auf und von der SIM-Karte) muß von der zentralen Steuereinrichtung 5 des externen Geräts übernommen werden. Die voreingestellte Rufummer wird auf der SIM-Karte zur Sicherheit auf einem anderen Speicherplatz permanent gehalten und benutzt, wenn das externe Gerät 2 mit einer neuen Rufnummer keine Verbindung aufbauen kann. Auf diese Weise wird sichergestellt, daß eine Kontaktaufnahme stets möglich bleibt.

Es können auch mehrere Servicezentralen betrieben werden. Der Patient meldet sich bei einer beliebigen Servicezentrale an. Dadurch ist dort die Adresse des Kunden bekannt und es kann entschieden werden, welche Servicezentrale zunächst den Dienst übernimmt. Schaltet der Kunde sein externes Gerät ein, dann meldet es sich bei der voreingestellten Servicezentrale an, indem es sofort eine SMS-Nachricht an diese abschickt. Die Servicezentrale kann nun durch Rücksendung einer SMS-Nachricht die Rufnummer übertragen, die in Zukunft verwendet werden soll. Das Mobilteil 3 kann so jederzeit automatisch einer anderen Servicezentrale zugeordnet werden, ohne daß ein Servicetechniker das Mobilteil 3 umstellen müßte. Dies macht besonders Sinn, wenn dadurch vermieden werden kann, daß SMS-Nachrichten über Grenzen geschickt werden müssen.

Die Datenbanken der Servicezentralen gleichen sich untereinander ab, so daß stets klar ist, welches Implantat 1 bzw. Mobilteil 3 von welcher Servicezentrale bedient wird. In der Datenbank sind entsprechende Felder vorzusehen.

Im Betrieb kann jeweils nur eine weitere (zweite) Nummer angenommen werden. Diese wird verwendet, wenn der Kontakt zu der "neuen" Servicezentrale erfolgreich hergestellt werden kann. Ist dies nicht der Fall, wird wieder die "alte" Rufnummer verwendet. Wird das externe Gerät erneut an eine weitere Servicezentrale überwiesen, wird die zweite Nummer überschrieben, so daß der Ablauf immer so ist, daß zunächst die zweite Nummer versucht wird und bei einem oder mehreren Fehlversuch(en) die erste Nummer benutzt wird. Der Vorteil dieses Verfahrens liegt darin, daß eine volle Flexibilität möglich ist, die sichere Rufnummer aber gespeichert bleibt, so daß ein völliger Zusammenbruch der Verbindung sicher vermieden wird.

In der SIM-Karte kann auch eine Art LiFo-Speicher für eine maximale Speichertiefe (z. B. zehn Rufnummern) organisiert werden. Gibt die bisher zuständigen Servicezentrale eine neue Rufnummer vor, wird diese als aktuellste Rufnummer verwendet. Entstehen aber mit dieser neuen Rufnummer Übertragungsprobleme, wird jeweils die nächst ältere Rufnummer verwendet, bis schließlich die permanent abgelegte voreingestellte Rufnummer an der Reihe ist. Ist der Speicher belegt, z.B. mit 9 Rufnummern und der voreingestellten Rufnummer, wird die älteste, also die Rufnummer, die der voreingestellten am nächsten liegt, gelöscht. Der Vorteil dieses Verfahrens ist seine hohe Redundanz, da anzunehmen ist, daß mit wenigstens einer der gespeicherten Servicezentralen eine Verbindung zustande kommt. Der Nachteil ist, daß unter Umständen viele Servicezentralen Daten von einem externen Gerät erhalten, die dann erst zusammengestellt und gemeinsam ausgewertet werden können.

Da das externe Gerät 2 mit einem Modem 12 ausgerüstet ist, kann der Rufnummernspeicher in der SIM-Karte zusätzlich auch für diese Rufnummern eingesetzt werden. Die Organisation kann man in Anlehnung an die Verwaltung der Mobilfunknummern gestalten. Durch das Abspeichern der Rufnummern auf der SIM-Karte, kann bei Austausch des externen Geräts beim Kunden, z.B. bei einem Defekt, die SIM-Karte wieder in das neue Gerät gesteckt werden, so daß die Art des Verbindungsaufbaus zu den Servicezentralen lückenlos übernommen wird.

### Basisstation 4

Die Basisstation 4 besteht aus einem Ladegerät 11 für die Akkus im Stromversorgungsteil 10 und einem als Datenübertragungseinrichtung wirkenden Modem 12 für das Telekommunikationsfestnetz 13, das vom Mobilteil 3 über die Schnittstelleneinrichtung 14 angesprochen wird. Die Basisstation 4 umfaßt weiterhin eine Zusatzantenne 15, die bei Verbindung mit dem Mobilteil 3 mit der Mobilfunkeinrichtung 7 verbunden ist und so die Übertragungsqualität verbessert.

Das Ladegerät 11 dient der Aufladung der Li-Ionen-Akkus des Mobilteils 3 und gleichzeitig der Versorgung der Komponenten der Basisstation 4. Das Ladegerät ist mit einem für Li-Ionenakkus geeigneten Regler und einer Überwachungsschaltung ausgerüstet. Es ist für einen maximalen Ladestrom von 350mA ausgelegt. Es ist umschaltbar für eine primäre Wechselspannung von 220/110V, 50/60Hz dimensioniert, wobei ein Bereich von 100 bis 240 V für die primäre Wechselspannung und ein Frequenzbereich von 47-63 Hz bevorzugt ist. Es wird Schutzklasse 2 für das externe Gerät 2 verwendet. Die Stromversorgung in der Basisstation 4 kann auch durch ein geeignetes Steckernetzteil ersetzt werden.

Die Schnittstelleneinrichtung 14 ist als Infrarotschnittstelle (IrDA) ausgebildet.

Die Zusatzantenne 15 ist als Diversity-Antenne ausgeführt. Es sind jeweils geeignete λ/4-Antennen vorgesehen, die als Loop- oder Helix-Antennen ausgeführt sind.

Es ist kein Netzschalter vorgesehen. Will man die Basisstation 4 vom Netz trennen, kann das Steckernetzteil gezogen werden.

### Externes Gerät 2: Zusammenwirken von Mobilteil 3 und Basisstation 4

Das Mobilteil 3 wird zum Laden und zur Datenübertragung über das Telekommunikationsfestnetz 13 in die Basisstation 4 eingesteckt.

Die Basisstation 4 ist mit einer Leuchtdiode ausgestattet, die aufleuchtet, wenn das Mobilteil 3 eingesteckt ist und ein Ladestrom die Akkus auflädt. Die Leuchtdiode erlischt, wenn das Mobilteil 3 entnommen bzw. kein Strom mehr vom Mobilteil 3 aufgenommen wird. Die Anzeige der Leuchtdiode wird für die Dauer der Übertragung, aber mindestens eine Sekunde invertiert, wenn über das eingebaute Modem 12 eine Nachricht verschickt oder empfangen wird. Die Basisstation 4 quittiert das Einlegen des Mobilteils 3 oder das Entnehmen des Mobilteils 3 mit einem kurzen Ton eines Buzzers.

Das Mobilteil 3 ist wahrend des Ladens in der Basisstation 4 voll funktionsfähig. Die Spannungswandler in der Basisstation sind bis 40 V ausgelegt, so daß ein Anschluß zum Laden aus einem Kfz-Netz möglich ist. Die Steuereinrichtung 5 überwacht die Akkuspannung und die Spannungsversorgung am Mobilfunkmodul 7.1. Bei eingeschaltetem Mobilteil 3 sind die Akkus nach ca. 4h in der Basisstation wieder voll aufgeladen.

Zur Datenübertragung ist die Steuereinrichtung 5 so ausgebildet, daß erst nach einer vorgegebenen Anzahl erfolgloser Übertragungsversuche über die Mobilfunkeinrichtung 7 die Übertragung der Daten an die Überwachungseinrichtung über das Telekommunikationsfestnetz 13 mittels der Schnittstelleneinrichtungen 8 und 14 und des Modems 12 erfolgt. Falls also die Mobilfunkübertragung nicht funktioniert oder das externe Gerät 2 in einem Land betrieben wird, das keinen Mobilfunkstandard unterstützt, d.h. liegen im Mobilteil 3, Daten vor, die im Speicher der SIM-Karte 7.2 zwischengespeichert und nicht über das Mobilfunknetz an die Servicezentrale übertragen werden konnten, erfolgt die Übertragung über das Modem, sobald das Mobilteil in die Basisstation gesteckt wird.

Die IR-Schnittstelle 8 des Mobilteils 3 und die IR-Schnittstelle 14 der Basisstation 4 sind dauernd empfangsbereit. Das Mobilteil 3 tauscht mit der Basisstation 4 Daten aus, sobald es in diese eingesteckt ist. Damit ist dem Mobilteil 3 jeweils bekannt, ob ein funktionsfähiges Modem 12 in der Basisstation 4 vorhanden ist.

Da es sich bei den zu übertragenden Nachrichten um sehr kurze Datenblöcke handelt, ist eine Übertragungsgeschwindigkeit 9600 Baud ausreichend. Das Modem 12 ist entweder in der Basisstation 4 integriert oder bei Bedarf durch den Servicetechniker anschließbar, indem es einfach in die Basisstation 4 eingeschnappt wird. Das Modem 12 ist auf einen Softwarehandshake umschaltbar, da über die IrDA-Schnittstellen 8 und 14 nur diese Art der "optischen 2-Draht-Übertragung" unterstützt wird.

### Externes Gerät 2: Selbsttest

Wird ein Fehler im Betrieb erkannt, eine entsprechende Anfrage der Servicezentrale empfangen oder geht das externe Gerät 2 nach einem Power-On-Reset wieder ans Netz, führt das externe Gerät 2 einen Selbsttest durch und meldet das Ergebnis an die Servicezentrale. Das externe Gerät 2 meldet also selbsttätig Fehler, läßt sich von der Servicezentrale aus prüfen und meldet sich zurück, wenn es wieder betriebsbereit ist (z.B. nach Wiederaufladen der entladenen Akkus). Der SMS-String mit dem Ergebnis des Selbsttests wird nur übertragen, wenn obige drei Vorbedingungen dies erfordern, im normalen Übertragungsstring ist keine Information zum Selbsttest enthalten.

Der Selbsttest ist dazu da, Rückschlüsse auf die Funktion der Hardware zuzulassen. Es kann dann im Servicezentrum entschieden werden, wie diese Fehler behandelt werden müssen. Das externe Gerät 2 meldet sich in sechs Fällen sofort über SMS-Nachrichten, ersatzweise über Modem:
- Ein gefährlicher Zustand wird vom Schrittmacher gemeldet.
- Die Übertragungsstrecke zum Schrittmacher funktioniert nicht.
- Ein Selbsttest des externen Geräts meldet einen Fehler.
- Bei Meldung eines Low-Battery-Zustandes vor dem Abschalten.
- Bei Patientenalarm, d.h. der Patient löst durch Drücken einer Taste des Interfaces 9 Alarm aus.
- Auf Anforderung durch die Servicezentrale.

Als Möglichkeiten der Selbstdiagnose des externen Gerätes können folgende Kriterien herangezogen werden:
a) Die Prüfsumme über den Programmcode (Flash-ROM-, EEPROM-, ROM-Fehler, Programm fehlerhaft geladen) ergab einen Speicherfehler.
b) Ein Speichertest des RAM ergab einen Speicherfehler.
c) Ein temporärer Ausfall des Mobilfunknetzes ist aufgetreten (kann auch Hardwarefehler bedeuten; das Auslesen der Mobilfunkeinrichtung 7 kann hier Klarheit schaffen).
d) Ein Timeout bei der Kommunikation mit der Servicezentrale ist aufgetreten (wie c).
e) Ein empfangener Datenstring ist zu kurz oder Prüfsummenfehler ist aufgetreten (Hardwarefehler in der Telemetrie oder Empfangsstörung bzw. Reichweiteprobleme).

### Externes Gerät 2: Zeitsynchronisation

Für die Mikroprozessoren in externen Geräten ist mit ihrer Real-Time-Clock nur die relative Zeit verfügbar. Die Zeit im Implantat 1 ist nicht justiert und nach einem halben Jahr auf + /-0,5 Stunden genau, so daß sie zur Festlegung der Systemzeit des externen Gerätes 2 nicht verwendet werden kann. Wenn beim Mobilteil 3 der Akku leer wird oder durch den Servicetechniker entnommen wird, kann es dazu kommen, daß die Real-Time-Clock die Zeit völlig verliert. Das externe Gerät benötigt aber die aktuelle Zeit zur Angabe in Verbindung mit Daten, die an die Servicezentrale verschickt werden, um zu dokumentieren, wann Ereignisse vom Implantat 1 empfangen wurden.

Die Daten werden als SMS-Nachrichten versandt, eingegangene Nachrichten werden durch die Servicezentrale über SMS gegenüber dem externen Gerät 2 quittiert. Um die aktuelle Zeit im externen Gerät 2 einzustellen, d. h. eine Zeitsynchronisation des externen Geräts 2 durchzuführen, wird die Zeitsignatur (SMS-Header) des Quittungs-SMS verwendet, die Datum und Uhrzeit enthält.

Kommt diese Quittungs-SMS innerhalb von z.B. einer Minute zurück kann das externe Gerät 2 die absolute Zeit nach dem Header der Quittung einstellen, da gewährleistet ist, daß beide SMS weniger als eine Minute unterwegs waren. Ist zwischen gesendeter SMS und der Quittung eine längere Zeit vergangen, kann der SMS-Header der Quittung nicht ausgewertet werden, da nicht festgestellt werden kann, ob die Originalsendung oder die Quittung verzögert übertragen wurde. Die Zeit für eine SMS-Übertragung liegt erfahrungsgemäß im Bereich unter 10s, so daß man obiges Verfahren gut anwenden kann.

Da die Wahrscheinlichkeit sehr hoch ist, daß nach einem Power-On-Reset die Uhrzeit im externen Gerät 2 falsch ist, ist für diesen Fall eine sofortige Zeitsynchronisation vorgesehen. Wenn das externe Gerät 2 nach einem Power-On-Reset wieder ans Netz geht, meldet es dies sofort via SMS an die Servicezentrale. Mit der SMS-Quittung von der Servicezentrale kann dann sofort die Uhr des externen Geräts gestellt werden.

Es kann festgelegt werden, daß sowohl die Servicezentrale, als auch das externe Gerät 2 mit der UTC-Zeit betrieben werden. Damit arbeitet das ganze System auf einer Zeit. Die Ortszeit wie z.B. MEZ oder MESZ muß dann bei Erstellung von Arztbriefen aus der UTC-Zeit ermittelt werden.

Sollte es bei der Ermittlung der absoluten Zeit Probleme geben, kann als Alternative noch die Zeitübertragung über das Modem 12 verwendet werden. Ein solcher Dienst wird in der Regel von verschiedenen Anbietern für das öffentliche Telefonnetz zur Verfügung gestellt.

### Externes Gerät 2: Service-Schnittstelle

Über die Schnittstelleneinrichtung 16 kann beispielsweise ein Servicetechniker unter Verwendung z.B. eines Laptops mit dem Mobilteil 3 kommunizieren. Eine andere Möglichkeit ist, daß das externe Gerät als Data-Logger arbeitet, indem Daten (z.B. als Blutzuckermonitor) eingelesen werden, die dann ebenfalls via SMS an die Servicezentrale geschickt werden.

Die Reichweite der Schnittstelleneinrichtung 16 beträgt wenigstens 0,4m. Sie ist so angeordnet, daß sie von einem Gerät, das neben dem externen Gerät 2 steht, benutzt werden kann, wenn das Mobilteil 3 in die Basisstation 4 eingelegt ist. Sie arbeitet bidirektional und kann im halb-duplex-Betrieb Daten übertragen. Die Übertragungsgeschwindigkeit der dritte Schnittstelleneinrichtung 16 beträgt wenigstens 9600 Baud und kann dann bis auf 115 KBaud hochgeschaltet werden.

Die dritte Schnittstelleneinrichtung 16 ist so anzuordnen, daß die Kommunikation zwischen Basisstation 4 und Mobilteil 3 sicher funktioniert, d.h. Beeinflussungen der zweiten Schnittstelleneinrichtung 8 durch die dritte Schnittstelleneinrichtung 16 ausgeschlossen sind.

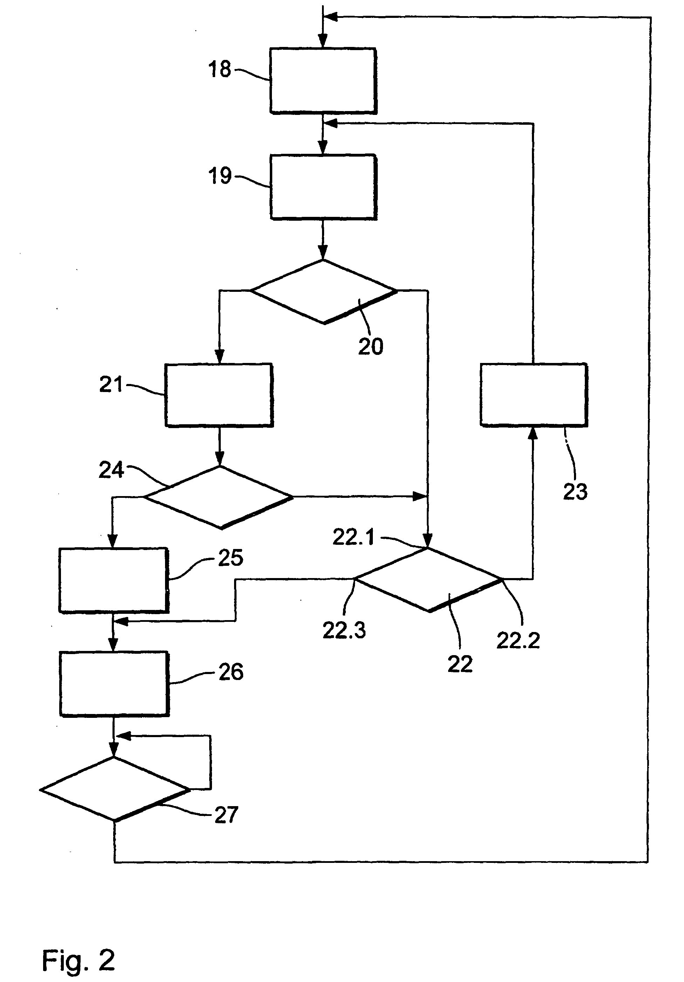

Figur 2 zeigt einen Ablaufplan eines in Verbindung mit dem erfindungsgemäßen Verfahren anwendbaren Verfahrens zur Datenübertragung, das zur Datenübertragung zwischen dem Implantat 1 und dem externen Gerät 2 verwendet werden kann.

Das Auslösesignal zur Datenübertragung wird dabei stets von der Sender/ Empfängereinheit des Implantats 1 ausgesandt, wozu in einem Schritt 18 die Sender/Empfängereinheit eingeschaltet wird.

In Schritt 19 erfolgt dann die Übertragung der ersten Daten aus dem Implantat 1 an das externe Gerät 2. Es wird ein String übertragen, der aus 17 Byte besteht. Dies sind 1 Byte Synchronisation, 4 Byte Identifikation, 1 Byte laufende Nummer der Übertragung, 1 Byte Länge des übertragenden Strings, 8 Byte Daten, 2 Byte Security (CRC). Bei asynchroner Übertragung ergibt sich ein Overhead von 3 Bit je Byte, so daß 17* 11 Bit = 187 Bit übertragen werden müssen.

In Schritt 20 erfolgt daraufhin eine Plausibilitätsprüfung der übersandten ersten Daten durch das externe Gerät. Hierbei erfolgt beispielsweise eine Kontrolle der CRC-Bits im Bezug auf den gesendeten String oder eine Überprüfung, ob die Synchronisationsbits die vorher bekannten Werte aufweisen. Weiterhin kann überprüft werden, ob die Stringlänge mit der gesendeten Stringlänge übereinstimmt und/oder ob die laufende Nummer der Übertragung korrekt ist. Eine Überprüfung von Flags entfällt, da im externen Gerät 2 kein Handling der Flags erfolgt. Bei Erweiterungen der Telemetrie kann somit immer der gleiche Typ eines externen Geräts verwendet werden. Eine Überprüfung der Implantatnummer entfällt, da das externe Gerät die Information mehrerer Implantate weiterleiten kann.

Erkennt das externe Gerät in Schritt 20, daß die Daten nicht plausibel sind, wird in Schritt 22 festgestellt ob eine vorgegebene Anzahl erneuter Übertragungsversuche überschritten wurde.

Wird in Schritt 22 festgestellt, daß die vorgegebene Anzahl erneuter Übertragungsversuche nicht überschritten wurde, werden in Schritt 23 erneut gültige Daten vom Schrittmacher angefordert. Diese Anfrage kann aus Synchronisation (8 Bit), ldentifikation (32 Bit), der laufenden Nummer der Nachricht (8 Bit) und einem vereinbarten Code (8 Bit) bestehen. Diesen String kann noch mit einem CRC-Byte abgesichert sein. Es werden dann 64 Bit + 8 * 3 Bit = 88 Bit gesendet, um eine Sendeabfrage des Schrittmachers einzuleiten.

Erkennt das externe Gerät in Schritt 20, daß die Daten plausibel sind, wird in Schritt 21 nach der erfolgten Plausibilitätsprüfung eine erste Quittung an das Implantat 1 gesendet, wobei die erste Quittung eine erste Steuerinformation zur Steuerung der Empfangsbereitschaft der erste Sender/Empfängereinheit enthält. Falls der Datensatz als gültig erkannt wurde, wird hierbei mit oder in der ersten Quittung ein Kontrolldatensatz übersandt, der durch das Implantat 1 kontrolliert wird. Hierbei gibt es zwei Möglichkeiten:

Als sicherste Methode wird der zuvor gesandte erste Datensatz komplett an das Implantat 1 zurückgeschickt, das dann den abgeschickten mit dem empfangenen Datensatz vergleicht. Es würden dann erneut 187 Bit zurückgeschickt.

Alternativ werden die Synchronisation (8 Bit), Identifikation (32 Bit), die laufende Nummer der Übertragung (8 Bit) und die CRC-Bits (16 Bit) zur Kontrolle an das Implantat 1 zurückgeschickt, das dann prüft, ob der gesendete String (Identifikation, Nummer der Übertragung) und ob die empfangenen CRC-Bits mit den CRC-Bits des gesendeten Nachricht übereinstimmen. Es werden dann 8 statt 17 Byte wie oben, d.h. es sind 64 Bit + 8 * 3 Bit = 88 Bit übertragen (etwa die Hälfte bezogen auf Variante 1).

Fällt in Schritt 24 die Kontrolle der ersten Quittung im Implantat negativ aus, d.h. sind die Kontrolldaten nicht identisch mit den Ausgangsdaten, erfolgt die Sendung des ersten Datensatzes erneut. Das Implantat versucht es dann nach einer festgelegten Zeitspanne nochmals. Das externe Gerät 2 informiert die Servicezentrale über die Anzahl falsch übertragener Datenpakete.

Fällt in Schritt 24 die Kontrolle der ersten Quittung im Implantat positiv aus, sendet das Implantat in Schritt 25 eine zweite Quittung an das externe Gerät 2. Diese zweite Quittung kann aus Synchronisation (8 Bit), Identifikation (32 Bit), der laufenden Nummer der Nachricht (8 Bit) und einem vereinbarten Code (8 Bit) bestehen. Diesen String kann noch mit einem CRC-Byte abgesichert sein. Es werden 64 Bit + 8 * 3 Bit = 88 Bit gesendet.

In Schritt 26 stellt das Implantat 1 dann alle Sende- und Empfangsaktivitäten ein.

In Schritt 27 wird der Ablauf eines bestimmten Zeitintervalls überprüft, nach dem ein neuer Datensatz im Implantat zur Sendung ansteht.

Nach dem Empfang der zweiten Quittung wird das Mobilteil 3 die nun verifizierten ersten Daten an die Servicezentrale weiterleiten. Der erste Datensatz ist als gültig markiert und die Servicezentrale weiß, daß diese Daten mit einer sehr hohen Wahrscheinlichkeit richtig sind. Das externe Gerät behält diese ersten Daten, bis das Implantat einen neuen, als richtig verifizierten Datensatz abliefert.

Falls beim Empfang der zweiten Quittung ein Fehler auftritt (z.B. andere lmplantatnummer, oder Quittungscode stimmt nicht) werden die ersten Daten trotzdem per SMS an die Servicezentrale gesandt, es wird aber zusätzlich vom externen Gerät 2 gemeldet, daß die zweite Quittung des Implantats 1 nicht gültig übertragen werden konnte.

In diesem Fall besteht die Möglichkeit, daß das Implantat 1 nach Absendung der zweiten Quittung nach Ablauf eines genau festgelegten Zeitintervalls wieder auf Empfang geht. Bei einer erneuten Anfrage kann das externen Gerät 2 beispielsweise die erste Quittung aus 88 Bit erneut schicken, um eine neue Sendung anzufordern. Das Implantat 1 kann sofort, nachdem es geprüft hat, daß es angesprochen ist, mit der erneuten Sendung beginnen, ohne daß erst die empfangenen Daten ausgewertet werden müssen. Hierbei kann die zweite Quittung oder der erste Datensatz erneut gesendet werden. Das externe Gerät 2 hat dann entweder den ersten Datensatz oder die zweite Quittung doppelt vorliegen und kann nun entscheiden, ob der erste Datensatz als gültig angenommen werden kann.

Die Sende/Empfangszyklen können sich mehrmals wiederholen. Es muß aber aus Gründen der Energieersparnis darauf geachtet werden, daß nach einer bestimmten Anzahl von Sendungen abgebrochen wird, um den Energiespeicher des Implantats nicht unnötig zu entladen. Das externe Gerät sollte dann trotzdem die Daten an die Servicezentrale senden, die Daten müssen aber als nicht verifiziert markiert sein. Die Daten werden im externen Gerät 2 gehalten, bis ein neuer Datensatz vom Implantat gesendet wird. Zusätzlich sollte der letzte gültige Datensatz im externe Gerät gehalten werden, den dann die Servicezentrale abfragen kann. Bei mehreren Implantaten, die zusammen mit dem externen Gerät 2 arbeiten, sind die jeweils letzten Datensätze jedes Implantats im externen Gerät 2 zu halten.

Der Empfänger des externen Geräts 2 ist bei der beschriebenen Variante des Verfahrens jederzeit bereit, Daten vom Implantat zu empfangen.

Hinsichtlich anderer Varianten des Verfahrens ist folgendes anzumerken: Zu regelmäßig wiederkehrenden oder beliebigen Zeitpunkten werden Sendungen vom Implantat 1 zum externen Gerät 2 geschickt, die entweder Daten enthalten oder nur anzeigen, daß das Implantat 1 aktiv und empfangsbereit ist. Ist mehr als eine Nachricht zu versenden, so werden sie nacheinander versendet. Diese Sendungen werden von der externen Gegenstelle empfangen, analysiert und quittiert. Die Quittungen werden in einem festen Zeitfenster nach der Sendung erwartet, so daß der Empfänger im Implantat 1 nur kurze Zeit aktiv sein muß. In den Quittungen können bereits Daten zum Implantat 1 übertragen werden. Anhand des Inhalts und Typs der Quittungen wird angezeigt, ob im externen Gerät 2 weitere Daten zur Übertragung in Richtung Implantat 1 anstehen. Die Quittungen werden vom Implantat 1 jeweils daraufhin analysiert, ob die externe Gegenstelle 2 weitere Daten versenden will und es wird dann automatisch im Protokoll für die Übertragung von Daten in der Gegenrichtung (wie oben beschrieben) fortgefahren. Dieser Zyklus kann sich nun beliebig oft wiederholen, bis alle Daten in beide Richtungen übertragen worden sind und kein Gerät weitere Daten senden möchte.

In einer weiteren Variante wird auch eine Reduktion des Energieverbrauchs im externen Gerät 2 erzielt. Sobald eine Nachricht vom externen Gerät 2 empfangen worden ist, synchronisieren sich Implantat und externes Gerät durch Festlegung auf Zeitscheiben, wodurch periodische Zeiten definiert werden, in denen der Empfänger des externen Gerätes aktiv ist. Das Implantat 1 darf dann auch nur innerhalb dieser Zeitscheiben senden, um empfangen zu werden. Die obengenannten Handlungsanweisungen werden dementsprechend abgeändert, so daß das Implantat 1 nur innerhalb der Zeitscheiben senden kann und nicht mehr zu beliebigen Zeiten.

Für eine bestehende Kommunikation (Abfolge von Sendungen und Quittungen) gilt diese Einschränkung nicht, so daß der Empfänger im externen Gerät 2 nach dem Absetzen einer Quittung noch einige Zeit aktiv bleibt, um eine etwaige Folgesendung des Implantats 1 empfangen zu können. Das Implantat muß nicht in jeder Zeitscheibe auch eine Sendung absetzen. Wenn das externe Gerät 2 Kontakt zu mehreren Implantaten halten soll, so werden für jedes angemeldete Implantat eigene Zeitscheiben verwaltet, die sich nach Möglichkeit nicht überlappen sollten um Störungen bei gleichzeitigem Senden verschiedener Implantate zu vermeiden.

Die Zeitscheiben können im Rahmen der oben beschriebenen normalen bidirektionalen Kommunikation sowohl in der Dauer der aktiven als auch der passiven Phase als auch dem Synchronisationszeitpunkt verändert werden. Werden über einen bestimmten Zeitraum keine Sendungen mehr vom Implantat empfangen, so wird schrittweise die aktive Zeitscheibe auf Kosten der passiven verlängert, um ein Auseinanderdriften der Synchronität aufzufangen. Im Extremfall ist der Empfänger im externen Gerät dauernd aktiv. Dies ist auch der Reset-Zustand beim Einschalten.

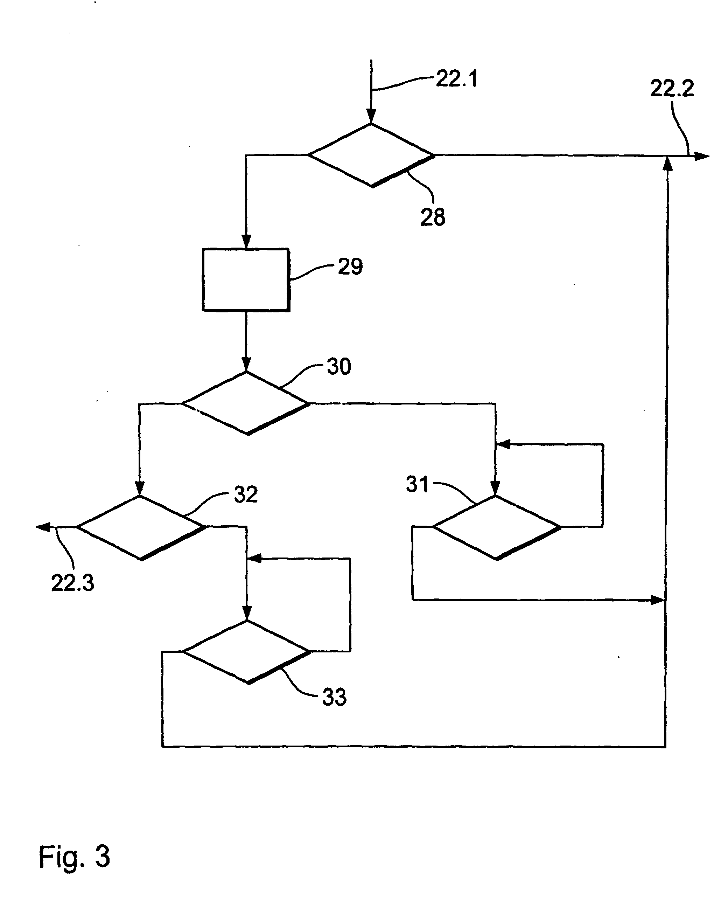

In Figur 3 ist als Ablaufdiagramm dargestellt, welche Strategie bei den Wiederholungen von Sendungen durch das Implantat 1 eingeschlagen werden kann. Figur 3 stellt Schritt 22 aus Figur 2 mit dem Eingang 22.1 und den Ausgängen 22.2 und 22.3 im Detail dar.

In Schritt 28 wird zunächst geprüft, ob ein Zähler Z einen vorgegebenen ersten Wert, hier 2, noch nicht überschritten hat. Der Zähler Z repräsentiert dabei die Anzahl der durchgeführten Übertragungsversuche. Er wird in Schritt 18 auf Null gesetzt und mit jedem Durchlaufen von Schritt 19 um 1 erhöht. Ist der vorgegebene erste Wert noch nicht überschritten, wird sofort über Ausgang 22.2 eine neue Übertragung initiiert. Zunächst wird also ein zweites Mal versucht, die Sendung zu übertragen.

Ist der vorgegebene erste Wert überschritten, so wird in Schritt 29 ein Timer auf Null gesetzt.

Dann wird in Schritt 30 geprüft, ob der Zähler Z einen vorgegebenen zweiten Wert, hier 3, noch nicht überschritten hat. Ist der vorgegebene zweite Wert noch nicht überschritten, wartet das System durch die Warteschleife 31. merklich, da von einem systematischen Fehler bei der Übertragung ausgegangen werden kann. Dies kann z.B. ein Störer sein, der für einige Zeit die Übertragung unmöglich macht (z.B. ein nicht ausreichend entstörtes Elektrogerät). Nach einer Wartezeit von z. B. 5 Minuten wird ein weiterer Sendeversuch gestartet. Ist dieser ebenfalls erfolglos, wird der Zyklus bei Schritt 28 fortgesetzt.

Dann wird in Schritt 32 geprüft, ob der Zähler Z einen vorgegebenen dritten Wert, hier 4, noch nicht überschritten hat. Ist der vorgegebene dritte Wert noch nicht überschritten, wartet das System durch die Warteschleife 33 noch länger, beispielsweise 1 Stunde, und startet einen letzten Sendeversuch. Ist dieser ebenfalls erfolglos, wird der Zyklus bei Schritt 28 fortgesetzt und danach bei 22.3 verlassen. Dann wird also erst wieder gesendet, wenn neue Daten im Schrittmacher zur Sendung bereitliegen.

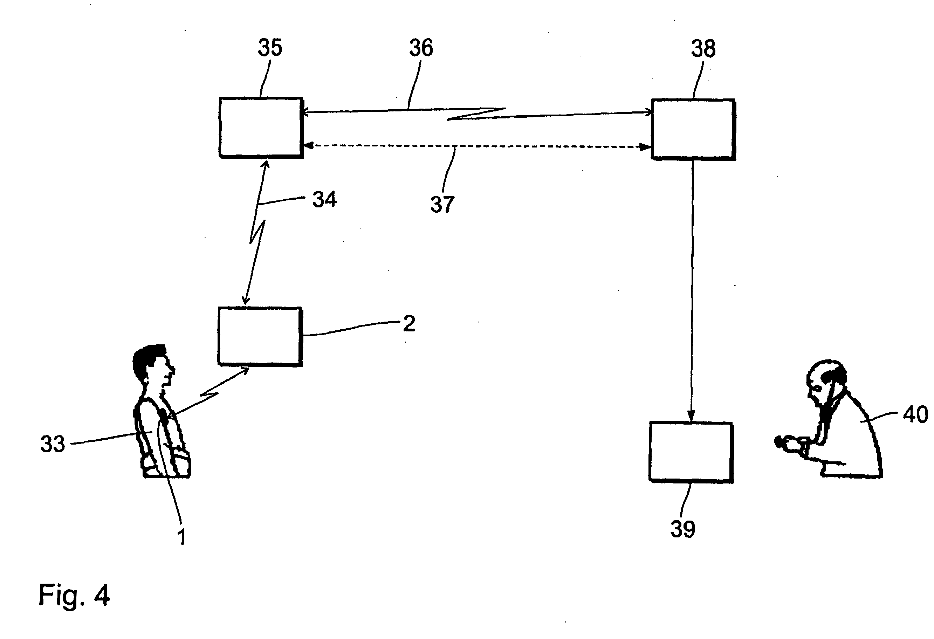

Figur 4 zeigt ein Blockschaltbild einer Variante eines Patientenüberwachungssystems unter Verwendung der vorliegenden Erfindung.

Dabei erfolgt eine Datenübertragung zwischen dem Implantat 1 und dem externen Gerät 2 aus Figur 1, das Implantat 1 wird dabei von einem Patienten 33 getragen. Von dem externen Gerät 2 werden die ausgelesenen Daten über eine Mobilfunkstrecke 34 eines Mobilfunknetzes an eine Mobilfunkstation 35 des Mobilfunknetzes übertragen und von dort ebenfalls über eine Mobilfunkstrecke 36 oder alternativ über eine Festnetzverbindung 37 an eine Servicezentrale 38 versandt, die als zentrale Speichereinrichtung und Überwachungseinrichtung dient. Alle Daten werden dabei als SMS-Nachrichten versandt und gegenseitig über SMS quittiert.

Die zentrale Speichereinheit 38 ist mit einer vollautomatischen Datenbank ausgestattet. Die medizinischen Daten werden automatisch über TCP/IP-Verbindung in den Datenbank-Rechner eingelesen, dort werden sie je nach Zeitpunkt, Implantat, Patient, externem Gerät und medizinischem Ansprechpartner (Arzt/Klinik) in die vorgesehenen Tabellen und Tabellenfelder eingetragen.

Bei Erreichen eines gewissen Füllegrades der Datenbank werden die Daten vom Rechner auf externe Medien (z.B. CD, Band) ausgelagert. Gemäß den medizinischen Richtlinien für Datensicherheit werden patientenbezogene Daten sofort (im Rahmen der Möglichkeiten) auf externe Read-Only-Medien (CD-ROM) gesichert. Ein geordneter Zugriff auf alle extern gesicherten Daten ist ständig über entsprechende Verweise in der Datenbank möglich.

Die Zugriffsmöglichkeiten auf die Daten werden geregelt durch komplexe Rechteverteilung (z.B. Lesen, Schreiben, Verändern, Löschen, etc.) für verschiedene Benutzergruppen wie Datenverwalter, Klinik oder Arzt. Während des Betriebs der Datenbank sind jederzeit manuelle Eingriffe und Abfragen durch Systembetreuer und andere Klienten (z.B. den Arzt 40) möglich.

Prioritätsgesteuerte Trigger in der Datenbank und ein angekoppeltes Expertensystem ermöglichen es, gezielt auf medizinische Sachverhalte zu reagieren: Die Datenbank generiert entsprechend vordefinierter Regeln Informationen für den Arzt und den Systembetreuer und gibt diese über Fax, E-Mail, interne und externe Rechnerverbindung an die Zielgeräte 39 weiter. Die ausgegebenen Informationen werden zur späteren Sammelabfrage in der Datenbank aufbewahrt. So kann der Arzt einen zusammenfassenden oder verlorenen Report bezüglich des Patienten erneut anfordern. Er erhält medizinische Informationen in wenigen Minuten auch während der Untersuchung und zusammengefaßte Informationen zum nächsten Nachsorgetermin. Das Übertragungsmedium und die Zieladressen sind dabei abhängig vom Auslösezeitpunkt und -Ereignis konfigurierbar. Dadurch kann der betreuende Arzt 40 auf eine von ihm gewünschte Art und Weise Informationen über den Gesundheitszustand seines Patienten 33 erhalten

Im Notfall kann der Patient 33 so auch schneller als üblich ärztliche Hilfe bekommen. Dieser Notfall wird sowohl durch objektiv gemessene medizinische Daten, als auch durch Tastendruck am Mobilteil 3 2 ausgelöst. Er erhält gegenüber den anderen Ereignissen höchste Priorität.

Weiter kann die Datenbank auch gesondert auf durch den Patienten ausgelöste Datenübertragungen reagieren, beispielsweise in dem auch in diesem Fall Informationen an den Arzt und den Systembetreuer gesendet werden. Dadurch kann das subjektive Befinden des Patienten in besonderem Maße bei der Datenerfassung berücksichtigt und in die Diagnose-Möglichkeiten miteinbezogen werden.

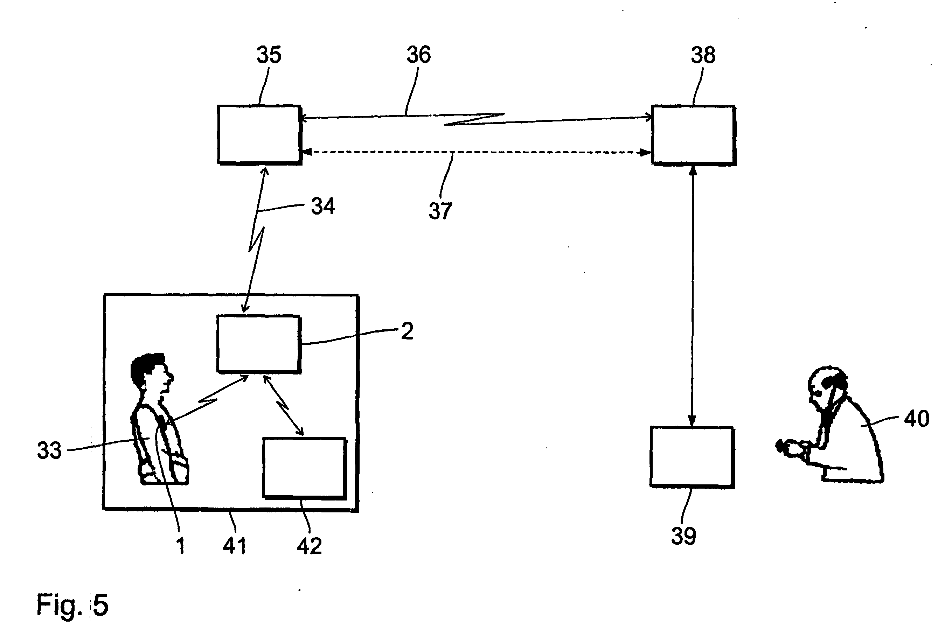

Figur 5 zeigt ein Blockschaltbild einer Variante eines Patientenüberwachungs- und - nachsorgesystems unter Verwendung der vorliegenden Erfindung.

Hierbei befindet sich der Patient 33 mit dem Implantat 1, und dem externen Gerät 2 zur Nachsorgeuntersuchung in einem Abfragebereich 41 einer Nachsorgeeinrichtung. Dabei wird, ausgelöst durch die mit dem externen Gerät bei betreten des Abfragebereichs 41 zusammenwirkende Auslöseeinrichtung automatisch eine Abfrage der ersten Daten aus dem Implantat 1 vor der Nachsorgeuntersuchung durch den Arzt 40 durchgeführt. Die Übertragung der ersten Daten erfolgt dabei mittels der langreichweitigen Telemetrieverbindung zwischen dem Implantat 1 und dem externen Gerät 2.

Das Ergebnis der Abfrage gelangt entsprechend aufbereitet über den zu Figur 4 beschriebenen Weg über die Servicezentrale 38 zum Zielgerät 39, von dem es an den Arzt 40 zu dessen Information ausgegeben wird. Gleichzeitig werden noch weitere patientenbezogene Daten aus der Datenbank der Servicezentrale 38 ausgegeben, so daß sich der Arzt 40 ein umfassendes Bild machen kann.

Die routinemäßige Momentan-Untersuchung des Arztes wird so durch ein Gesamtbild über die Zeit seit dem letzten Arztbesuch ergänzt. Der Arzt erhält ein Bild über mehrere Wochen oder Monate hinweg, was wesentlich aussagekräftiger ist als die momentane Untersuchung. Dabei werden Lebensgewohnheiten des Patienten gut abgebildet, wohingegen gerade der Termin beim Arzt eine Ausnahmesituation vom gewohnten Leben darstellen kann (Fahrt zur Klinik bzw. zum Arzt, Treppensteigen, Streß im Verkehr etc.).

Bei der eigentlichen Untersuchung kann der Arzt (z.B. bei Belastungstests) auch sofort einen Report aus der Servicezentrale 38 anfordern, der dann nach wenigen Minuten zugestellt wird. Hierdurch können Parameteränderungen, die mit Hilfe des Programmers eingestellt wurden, sofort überprüft und ggf. noch optimiert werden. Durch die Speicherung in der Datenbank und weitere Auswertemöglichkeiten durch das Expertensystem oder durch Vergleich mit anderen Fällen können weitere Reports erstellt werden. Durch Fortschritte in der medizinischen Erkenntnis können so neue Zusammenhänge erkannt werden. Diese werden dem Arzt dann beim nächsten Report mitgeteilt, ohne daß er bei neuen Erkenntnissen jeden seiner Patienten auf mögliche Auswirkungen überprüfen müßte.

In einer weiteren Variante der Erfindung ist es zusätzlich möglich, sobald der Patient 33 den Abfragebereich 41 einer Nachsorgeeinrichtung betritt, automatisch bestimmte standardmäßige Test- oder Diagnose-Programme zu starten, die normalerweise nur unter ärztlicher Aufsicht durchgeführt werden. Beispielsweise können Reizschwellen-, Sensitivitäts-, Belastungs- oder Defibrillations-Schock-Tests ausgeführt werden.

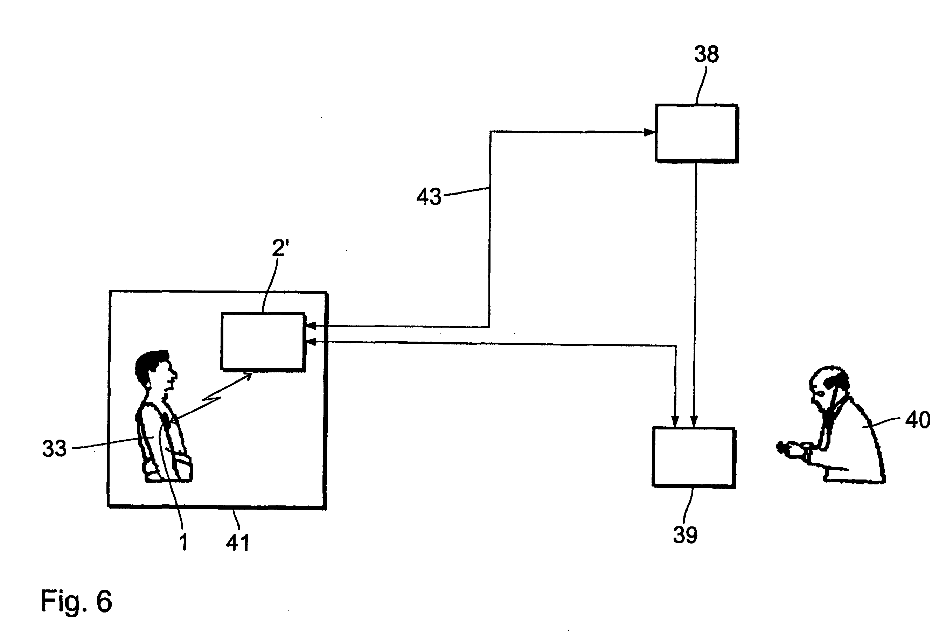

Figur 6 zeigt eine Variante der Ausführung aus Figur 5, bei der im Abfragebereich 41 der Nachsorgeeinrichtung ein externes Gerät 2' fest installiert ist. Hierbei wird die Abfrage ausgelöst, sobald sich der Patient 33 mit dem Implantat 1 für einen gewissen Zeitraum in Abfragereichweite des externen Geräts 2' befindet.

Das Ergebnis der Abfrage gelangt entsprechend aufbereitet direkt vom externen Gerät 2' zum Zielgerät 39, von dem es an den Arzt 40 zu dessen Information ausgegeben wird. Gleichzeitig werden über die Verbindung 43 noch weitere patientenbezogene Daten aus der Datenbank der Servicezentrale 38 abgefragt und am Zielgerät 39 ausgegeben, so daß sich der Arzt 40 auch hier ein umfassendes Bild machen kann.

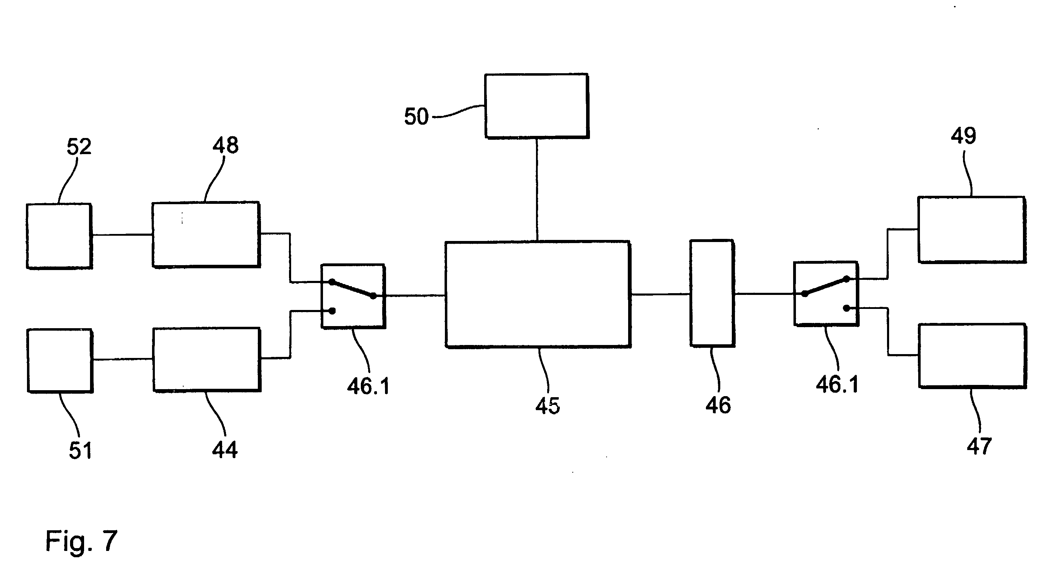

Figur 7 zeigt ein Blockschaltbild einer Telemetrieeinrichtung eines elektromedizinischen Implantats, die zusammen mit der vorliegenden Erfindung verwendet werden kann.

Die Telemetrieeinrichtung weist einen Nahfeldsender 44, eine damit verbundenen Telemetrieeinheit 45 und eine mit der Telemetrieeinheit 45 verbundene Antennenschnittstelleneinrichtung 46 auf, über welche eine Nahfeldantenne 47 mit der Nahfeld-Sender/Empfängereinheit verbunden ist.

Weiterhin ist zur Ausbildung einer Fernfeldtelemetrieeinrichtung ein mit der Telemetrieeinheit 45 verbundener Fernfeldsender 48 und eine mit der Antennenschnittstelleneinrichtung 46 verbundene Fernfeldantenne 49 vorgesehen.

Weiterhin weisen die Nahfeldtelemetrieeinrichtung und die Fernfeldtelemetrieeinrichtung getrennte Energiespeicher 51 und 52 auf.

Die übrigen notwendigen Systemkomponenten für eine Telemetrieeinrichtung, wie Zwischenspeicher, Ablaufsteuerung, Kodierung, Dekodierung und Treiber mit Schwellwertdetektor sind dabei in die Telemetrieeinheit 45 integriert.

Zum Umschalten zwischen Fernfeldsender 48 und Nahfeldsender 44 sind diese über eine Weiche 46.1 mit der Telemetrieeinheit 45 verbunden. Zum Umschalten zwischen Nahfeldantenne 47 und Fernfeldantenne 49 sind diese über eine weitere Weiche 46.2 mit der Antennenschnittstelleneinrichtung 45 verbunden. Es versteht sich jedoch, daß bei anderen Varianten die beiden Sender ebenso wie die beiden Antennen auch parallelgeschaltet an der Telemetrieeinheit 45 bzw. der Antennenschnittstelleneinrichtung 45 liegen können, da in der Regel Fern- und Nahfeldtelemetrie nicht gleichzeitig betrieben werden.

Zur Anpassung des Modulationsverfahrens für die jeweilige Telemetrie ist eine mit der Telemetrieeinheit 45 verbundene Anpassungslogikeinheit 50 vorgesehen. Die Fernfeldtelemetrieeinrichtung wird mit im wesentlichen derselben effektiven Datenrate wie die Nahfeldtelemetrieeinrichtung betrieben.

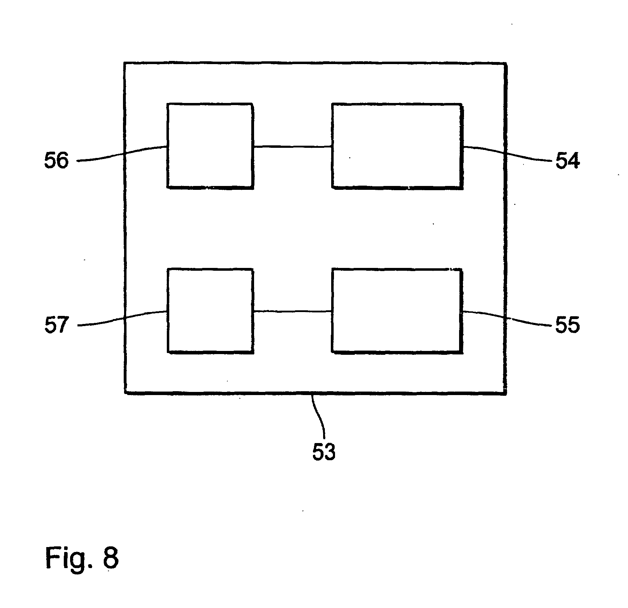

Figur 8 zeigt die Sender/Empfängereinheit einer Ausführung des Implantats 1, bei der für den Sender 54 und den Empfänger 55 getrennte Energiespeicher 56 und 57 vorgesehen sind. Dabei handelt es sich um separate Pufferkapazitäten 56 und 57. Diese Kapazitäten 56 und 57 müssen nur auf die für den jeweiligen Vorgang notwendige Energie geladen werden. Der Energieverbrauch des einen Vorgangs beeinflußt nicht den Energievorrat für den anderen Vorgang. Die Vorgänge können somit unmittelbar aufeinander folgen, was für ein bidirektionales Kommunikationsprotokoll von Vorteil ist, ohne daß hierfür die Aufladung einer einzigen Pufferkapazität auf den doppelten Energieinhalt erforderlich wäre.

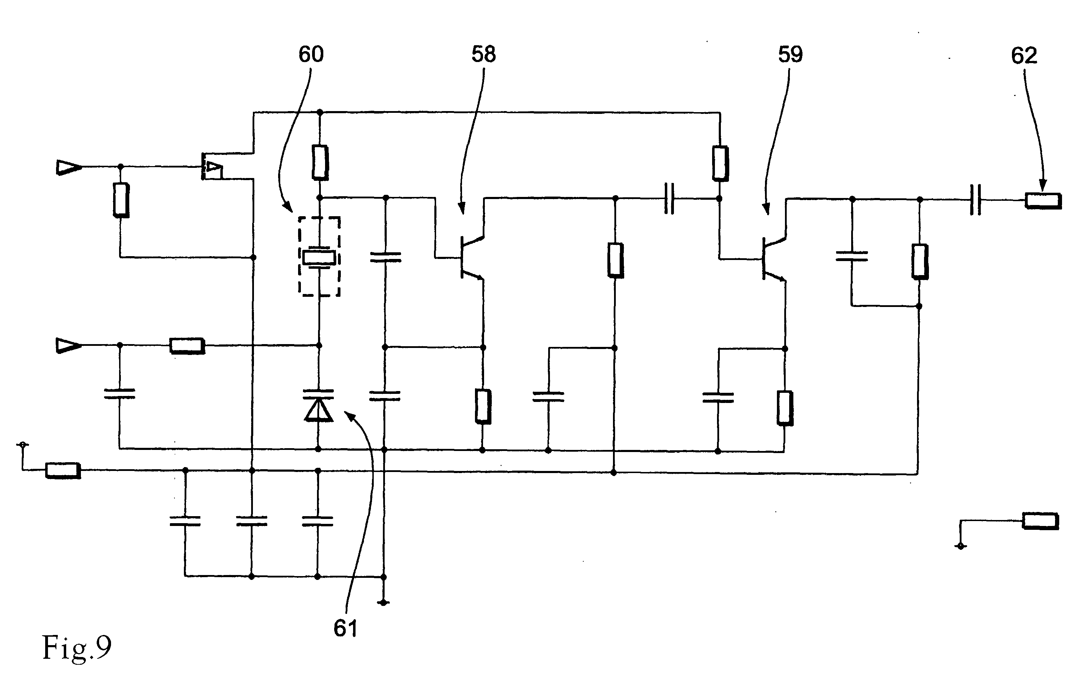

Figur 9 zeigt ein Schaltbild eines erfindungsgemäßen Senders der ersten Sender/Empfängereinheit einer Variante des Implantats 1 aus Figur 1.

Ein stabiler frequenzmodulierbarer Sender ist dabei mit zwei bipolaren Transistoren 58, 59 realisiert werden, wobei der erste Transistor 58 in einer Colpitts- oder Clapp-Schaltung mit einem SAW-Resonator 60 einen SAW-stabilisierten Oszillator bildet und der zweite Transistor 59 als Pufferstufe und Antennentreiber dient. Die Transistoren 58, 59 sind auf maximale Verstärkung bei geringstem Kollektorstrom ausgelegt. Mit einer Kapazitätsdiode 61 in Serie zum SAW-Resonator 60 kann die Frequenz moduliert werden. Der Frequenzhub, und damit die Datenrate und/oder die Reichweite des Senders, können jedoch dadurch erhöht werden, daß die Kapazitätsdiode durch eine PIN-Diode ersetzt wird, die von einem weiteren Transistor geschaltet wird.

Als Antenne kann eine einfache Drahtschleife oder ein offener Draht (Wurfantenne) in der Kontur des Headers des Implantats dienen. Insgesamt ist damit bei 400 MHz ein Stromverbrauch von weniger als 1 mA bei einer Reichweite von mehreren Metern möglich. Die Schaltung kann direkt aus einer von einer hochohmigen Batterie des Implantats 1 gespeisten Pufferkapazität oder einer niederohmigen Batterie versorgt werden. Eine Ladungspumpe ist nicht erforderlich.

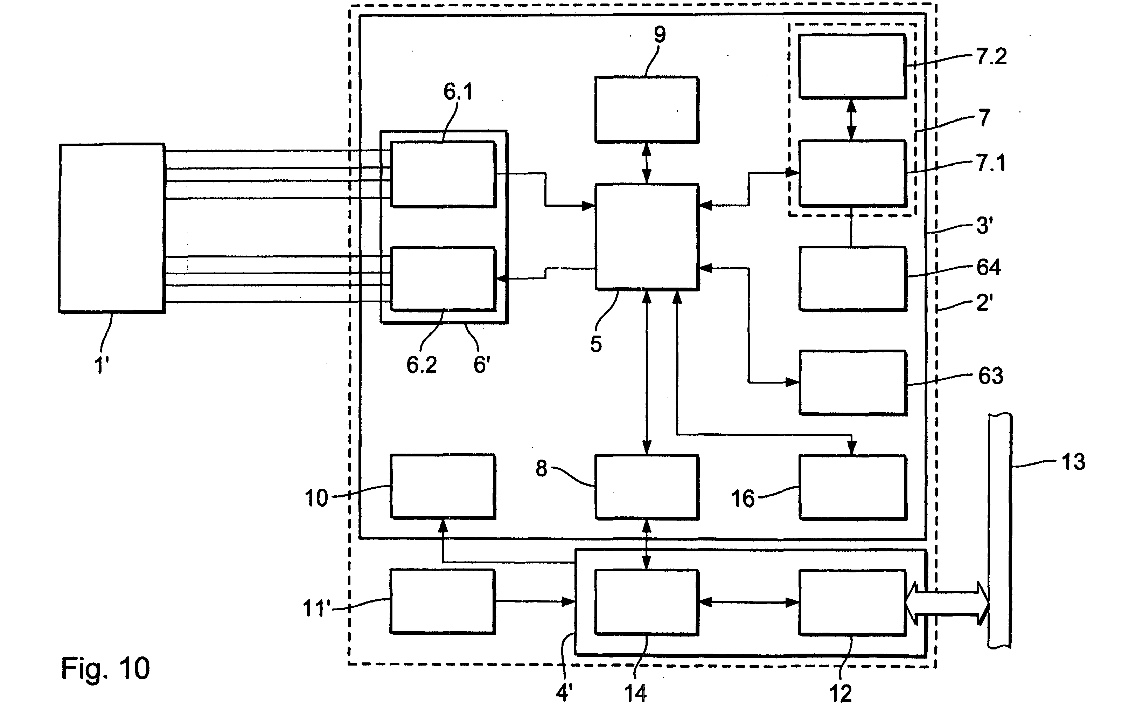

Figur 10 zeigt ein weiteres Ausführungsbeispiel einer erfindungsgemäßen Vorrichtung, die im wesentlichen derjenigen aus Figur 1 entspricht, weshalb hier lediglich auf die Unterschiede eingegangen werden soll.

Dabei handelt es sich um ein externes Gerät 2'', das sich zur Überwachung, Ansteuerung und Übertragung von Daten aus verschiedensten elektronischen Geräten 1' eignet. Anwendungen können sein die Fernüberwachung von Anlagen, z.B. des Füllgrades von Getränke- oder Verkaufsautomaten, die Anbindung an die Haustechnik, z.B. Steuerung und Überwachung der Klimaanlage oder Heizung, die Kopplung an Alarmanlagen oder Glasbruchmeldeanlagen, z.B. Einbruchmeldung via Mobilfunk, die Kopplung an Meßsysteme, z.B. Wetterstationen oder Pegelmesser an Flüssen, die Kopplung an Systeme zur Verkehrsbeobachtung und -beeinflussung, z.B. Schilderbrücken etc. Weiterhin kann es als Abhöranlage oder als Mobiltelefon mit digitalen Sonderfunktionen eingesetzt werden.

Die Unterschiede zur Ausführung aus Figur 1 betreffen im wesentlichen das Mobilteil 3' des externen Geräts 2''. Die erste Schnittstelleneinrichtung umfaßt dabei 4 potentialfreie Eingänge 6.1 über Optokoppler und 4 potentialfreie Ausgänge 6.2. Dank der Potentialtrennung der Ein- und Ausgänge 6.1 und 6.2 eignet sich das externe Gerät 2'' für die Überwachung und Steuerung von digitalen Zuständen.

Weiterhin entfällt der Empfangs- und Sendeteil 6 aus Figur 1, statt dessen wird die freiwerdende serielle Schnittstelle 63 (RS 232) über einen 9-poligen Stecker herausgeführt. Bei der RS 232 genügt ein Softwarehandshake mit drei Leitungen (RXD, TXD, GND). Damit ist die Übertragungsrate ebenfalls auf 9600 Baud begrenzt. Die serielle Schnittstelle 63 ist nicht potentialfrei ausgelegt. Über einem weiteren Stecker 64 können die Verbindungen des Mobilfunkmoduls 7.1 zu einem Mikrofon, einem Lautsprecher und optional einem Buzzer geführt werden (6 Leitungen), so daß für weitere Einsatzgebiete kann eine Hör/Sprechkombination und ein Buzzer hinzugefügt werden können.

Die Eingänge 6.1 liegen im Bereich 1 bei -3V bis 1,5V L-Pegel, 3,5 bis 8V H-Pegel und 10 mA Eingangsstrom; im Bereich 2 bei -3V bis 8V L-Pegel, 18V bis 30V H-Pegel und 10 mA Eingangsstrom. Die Zustandserkennung erfolgt über eine LED-Anzeige bei maximaler Schaltfrequenz 100Hz Rechteck und einer Trennspannung von 2,5kV.

Die Ausgänge weisen 15 bis 30V DC separate Spannungseinspeisung, einen Laststrom von 200mA, Kurzschlußschutz, Schutz gegen thermische Überlastung und eine Trennspannung von 2,5kV auf. Die Potentialtrennung wird über Optokoppler, die Leistungs-Schaltfunktion über MOS-FETs realisiert.

Ein Unterschied zur Ausführung aus Figur 1 bezüglich der Basisstation 4' besteht lediglich hinsichtlich der Stromversorgung durch ein Steckernetzteil 11' statt dem Netzteil in der Basisstation. Eine weitere Ausführungsmöglichkeit besteht darin, daß die Basisstation wegfällt und das externe Gerät direkt über ein Netzteil versorgt wird. Bei Netzausfall arbeitet das Mobilteil dann noch etwa 20 Stunden weiter.

Die Erfindung beschränkt sich in ihrer Ausführung nicht auf die vorstehend angegebenen bevorzugten Ausführungsbeispiele. Vielmehr ist eine Vielzahl von Varianten möglich, welche von der dargestellten Lösung auch bei grundsätzlich anders gearteten Ausführungen Gebrauch macht.

## Patentansprüche

1. Verfahren zur Nachsorge bei Patienten mit wenigstens einem elektromedizinischen Implantat (1), insbesondere einem Herzschrittmacher, bei dem
eine erste telemetrische Datenübertragung zwischen dem Implantat (1) und einem zugeordneten externen Gerät (2; 2') erfolgt, wobei Daten zwischen dem Implantat (1) und dem externen Gerät übertragen werden, und
zur Information des Untersuchenden anläßlich einer Nachsorgeuntersuchung des Patienten in einer Nachsorgeeinrichtung im Rahmen einer Nachsorgeabfrage wenigstens die aktuellen zur Verfügung stehenden Daten des Implantats (1) abgefragt und zur Ausgabe an entsprechende Ausgabemittel (39) gesandt werden,
**dadurch gekennzeichnet, daß** als externes Gerät ein im Abfragebereich installiertes externes Gerät (2') verwendet wird und
die im Rahmen der Nachsorgeabfrage vorgenommene Abfrage der Daten vor der Nachsorgeuntersuchung automatisch nach Eintreffen des Patienten in einem Abfragebereich (41) der Nachsorgeeinrichtung erfolgt, sobald sich das Implantat (1) für einen vorgegebenen Zeitraum in Abfragereichweite zum externen Gerät (2') befindet.

2. Verfahren nach Anspruch 1, **dadurch gekennzeichnet, daß** die Übertragung der Daten von dem Implantat zu dem externen Gerät über eine langreichweitige Telemetrieverbindung erfolgt

3. Verfahren nach Anspruch 1 oder 2, **dadurch gekennzeichnet, daß** die Übertragung dieser Daten von dem Implantat zu dem externen Gerät mindestens teilweise über ein Mobilfunknetz oder ein Telekommunikationsfestnetz erfolgt.

4. Verfahren nach einem der vorhergehenden Ansprüche, **dadurch gekennzeichnet, daß** zur weitergehenden Information des Untersuchenden neben der Abfrage der Daten vom Implantat automatisch wenigstens ein in einer zentralen Speichereinrichtung (38) gespeicherter, aus einer oder mehreren vorherigen Datenausgaben des Implantats (1) stammender Satz weiterer Daten abgerufen und zur Ausgabe an die Ausgabemittel (39) gesandt wird.

5. Verfahren nach Anspruch 4, **dadurch gekennzeichnet, daß** eine nach Art einer Datenbank organisierte zentrale Speichereinrichtung (38) verwendet wird.

6. Verfahren nach Anspruch 5, **dadurch gekennzeichnet, daß** die zentrale Speichereinrichtung (38) mit einem angekoppelten Expertensystem verwendet wird.

7. Verfahren nach einem der vorhergehenden Ansprüche, **dadurch gekennzeichnet, daß** vor der Ausgabe durch die Ausgabemittel (39) eine automatische Vorauswertung und/oder Aufbereitung der Daten vom Implantat und gegebenenfalls der weiteren Daten erfolgt.

8. Verfahren nach einem der vorstehenden Ansprüche, **dadurch gekennzeichnet, daß** das Auslösen der Abfrage der Daten durch eine im Abfragebereich (41) installierte erste Auslöseeinrichtung (42) erfolgt, die mit einer zugeordneten zweiten Auslöseeinrichtung des Implantats und/oder des mobilen externen Geräts zusammenwirkt.

9. Verfahren nach einem der vorstehenden Ansprüche, **dadurch gekennzeichnet, daß** zur weitergehenden Information des Untersuchenden Tests oder Diagnose-Diagramme in dem Implantat über die telemetrische Datenübertragung generiert werden, und die Ergebnisse über die telemetrische Datenübertragung an das externe Gerät übertragen werden.

## Claims

1. Method for the aftercare of patients with at least one electromedical implant (1), in particular a cardiac pacemaker, in which
a first telemetric data transmission is made between the implant (1) and an associated external device (2; 2'), whereby data is transmitted between the implant (1) and the external device, and
to provide an examining clinician with information on the occasion of an aftercare examination of the patient in an aftercare installation at least the current, available data on the implant (1) is requested in an aftercare enquiry procedure and sent for output to a corresponding output means (39),
**characterised in that** an external device (2') installed in the enquiry region is used as the external device, and
**in that** the request for data from before the aftercare examination carried out as part of the aftercare examination is performed automatically after the patient has arrived at an enquiry region (41) of the aftercare installation, as soon as the implant (1) is within the enquiry range of the external device (2') for a predetermined period of time.

2. Method according to claim 1, **characterised in that** the transmission of data from the implant to the external device is performed via a long-range telemetry connection.

3. Method according to claim 1 or claim 2, **characterised in that** the transmission of said data from the implant to the external device is performed at least partly via a mobile radio network or a fixed-line telecommunication network.

4. Method according to one of the preceding claims, **characterised in that** for the further information of the examining clinician in addition to the request for data from the implant at least one data record of additional data, which is stored in a central memory device (38) and originates from one or more previous data outputs from the implant (1), is requested automatically and sent for output to the output means (39).

5. Method according to claim 4, **characterised in that** a central memory device (38) is used that is organised in the manner of a database.

6. Method according to claim 5, **characterised in that** the central memory device (38) is used with an expert system coupled thereto.

7. Method according to one of the preceding claims, **characterised in that** prior to output via the output means (39) there is an automatic pre-evaluation and/or preparation of the data from the implant and if necessary of the additional data.

8. Method according to one of the preceding claims, **characterised in that** the request for data is initiated by a first actuating device (42) which is installed in the request region (41) and which cooperates with an associated second actuating device of the implant and/or the mobile external device.

9. Method according to one of the preceding claims, **characterised in that** to provide the examining clinician with further information tests or diagnosis diagrams are generated in the implant via the telemetric data transmission and the results are transmitted to the external device via the telemetric data transmission.

## Revendications

1. Procédé d'examen ou de soins post-cliniques chez des patients comportant au moins un implant électromédical (1), notamment un stimulateur cardiaque, selon lequel une première transmission télémétrique de données est effectuée entre l'implant (1) et un appareil externe associé (2;2'), des données étant transmises entre l'implant (1) et l'appareil externe, et
pour informer la personne pratiquant l'examen à l'occasion d'un examen post-clinique du patient dans un dispositif d'examen post-clinique dans le cadre d'une interrogation de maintenance, au moins les données actuellement disponibles de l'implant (1) sont interrogées et sont émises pour leur délivrance à des moyens correspondants de sortie (39),
**caractérisé en ce**
**qu'**on utilise comme appareil externe un appareil externe (2') installé dans la zone d'interrogation, et
**que** l'interrogation des données, exécutée dans le cadre de l'interrogation de maintenance, s'effectue automatiquement avant l'examen de maintenance, après que le patient a pénétré dans une zone d'interrogation (41) du dispositif d'examen post-clinique, dès que l'implant (1) est situé pendant un intervalle de temps prédéterminé dans une portée d'interrogation par rapport à l'appareil externe (2').

2. Procédé selon la revendication 1, **caractérisé en ce que** la transmission des données de l'implant à l'appareil externe s'effectue par l'intermédiaire d'une liaison télémétrique de longue portée.

3. Procédé selon la revendication 1 ou 2, **caractérisé en ce que** la transmission de ces données s'effectue de l'implant à l'appareil externe au moins en partie par l'intermédiaire d'un réseau audio mobile ou d'un réseau fixe de télécommunication.

4. Procédé selon l'une des revendications précédentes, **caractérisé en ce que** pour la poursuite de l'information fournie à la personne pratiquant l'examen, en dehors de l'interrogation des données de l'implant, automatiquement au moins un ensemble d'autres données, mémorisé dans un dispositif central de mémoire (38) et provenant d'une ou de plusieurs sorties précédentes de données de l'implant (1), est émis pour être délivré au niveau des moyens de sortie (39).

5. Procédé selon la revendication 4, **caractérisé en ce qu'**on utilise un dispositif central de mémoire (38) organisé à la manière d'une banque de données.

6. Procédé selon la revendication 5, **caractérisé en ce qu'**on utilise un dispositif central de mémoire (38) auquel est couplé un système expert.

7. Procédé selon l'une des revendications précédentes, **caractérisé en ce qu'**avant la délivrance par les moyens de sortie (39), une évaluation préalable et/ou un traitement automatique des données de l'implant et éventuellement des autres données est exécuté.

8. Procédé selon l'une des revendications précédentes, **caractérisé en ce que** le déclenchement de l'interaction des données est exécuté par un premier dispositif de déclenchement (42), installé dans la zone d'interrogation (41) et qui coopère avec un second dispositif de déclenchement associé de l'implant et/ou de l'appareil externe mobile.

9. Procédé selon l'une des revendications précédentes, **caractérisé en ce que** pour la poursuite de l'information fournie à la personne pratiquant l'examen, des tests ou des diagrammes de diagnostic sont générés dans l'implant au moyen de la transmission télémétrique de données, et les résultats sont transmis à l'appareil externe par l'intermédiaire de la transmission télémétrique de données.
